# EUROPÄISCHE PATENTSCHRIFT

(11) **EP 3 645 071 B1**
(45) Veröffentlichungstag und Bekanntmachung des Hinweises auf die Patenterteilung: **28.04.2021**
(21) Anmeldenummer: 18749723.5
(22) Anmeldetag: 27.06.2018
(51) Int. Cl.: A61M 1/16, A61M 1/28

(54) **KONZENTRATBEHÄLTNIS**
CONCENTRATE RECEPTACLE
RÉCIPIENT DE CONCENTRAT

(30) Priorität: 28.06.2017 DE 102017114400
(43) Veröffentlichungstag der Anmeldung: 06.05.2020
(73) Patentinhaber: Fresenius Medical Care Deutschland GmbH, 61352 Bad Homburg (DE)
(72) Erfinder: HEDMANN, Frank, 97332 Volkach (DE); SCHUBERT, Nadja, 97422 Schweinfurt (DE)
(74) Vertreter: Herrmann, Uwe
(86) Internationale Anmeldenummer: PCT/EP2018/067236
(87) Internationale Veröffentlichungsnummer: WO 2019/002361

(56) Entgegenhaltungen:
- EP-A1- 1 872 814
- EP-A2- 2 167 163
- EP-A2- 2 258 419
- DE-A1-102011 106 248

## Beschreibung

Die vorliegende Erfindung betrifft ein Konzentratbehältnis mit einem Konzentrat, das ausgebildet ist, nach seiner Verdünnung mit einem Verdünnungsmittel, insbesondere mit Wasser, eine Dialyselösung zu bilden.

In der Peritonealdialyse ist es üblich, vorgefertigte Dialyselösungen zu verabreichen. Diese werden typischerweise in Volumina von 3 bis 6 Litern verabreicht. Bei einer typischen Peritonealdialysebehandlung werden 3 Beutel pro Tag benötigt, was einen erheblichen logistischen Aufwand bedeutet und einen erheblichen Lagerplatz benötigt Konzentratbehältnisse für die Dialysatherstellung sind z.B. aus EP2258419 A2, EP2167163 A2 und EP1872814 A1 bekannt.

Der vorliegenden Erfindung liegt die Aufgabe zugrunde, ein Konzentratbehältnis bereitzustellen, das eine einfache Lösungsherstellung bei geringem Lagerbedarf ermöglicht.

Diese Aufgabe wird durch ein Konzentratbehältnis mit den Merkmalen des Anspruchs 1 gelöst.

Danach ist vorgesehen, dass das Konzentratbehältnis mit einem ersten Konnektor mit einem ersten Code in Verbindung steht, der wenigstens eine Information bzgl. des Konzentrats enthält, und dass das Konzentratbehältnis des Weiteren mit einem zweiten Konnektor in Verbindung steht, dessen zweiter Code sich von dem ersten Code unterscheidet, wobei der zweite Code wenigstens eine Information bzgl. der Dialyselösung enthält.

Ein derartiges Konzentratbehältnis benötigt wesentlich weniger Volumen als ein mit fertiger Dialyselösung gefülltes Behältnis. Das Konzentrat kann vor Ort, d.h. am PoC (Point of Care), wie z.B. zu Hause mit einem Verdünnungsmittel verdünnt werden, so dass aus dem Konzentrat eine Dialyselösung entsteht. An dieser Stelle wird darauf hingewiesen, dass unter dem Begriff "Dialyselösung" sowohl eine verabreichungsfertige Lösung zu verstehen ist als auch eine Lösung, die ggf. weiter verdünnt oder gemischt werden muss, um die fertige Dialyselösung zu erhalten.

Der erste Konnektor weist einen ersten Code und der zweite Konnektor weist einen sich davon unterscheidenden zweiten Code auf. Der erste Code enthält wenigstens eine das Konzentrat betreffende Information, wie z.B. die Menge, die Zusammensetzung bzw. den Konzentrattyp, die benötige Menge an Verdünnungsmittel etc. Der zweite Code enthält wenigstens eine die Dialyselösung betreffende Information, wie z.B. das Volumen, die Zusammensetzung, die Konzentrationen etc. der Dialyselösung.

Wird das Konzentrat verdünnt, wird zu diesem Zweck der erste Code ausgelesen, so dass das die Verdünnung vornehmende Gerät die Menge an Verdünnungsmittel etc. kennt und eine entsprechende Menge zugeben kann.

Wurde aus dem Konzentrat durch Verdünnung die Dialyselösung hergestellt, wird die Information des zweiten Codes ausgelesen, so dass beispielsweise bekannt ist, um welche Lösung es sich handelt.

Somit ist es möglich, mittels zweier Codes sowohl die Herstellung der Dialyselösung als auch die Verabreichung der hergestellten Dialyselösung vorzunehmen, wobei die Codes vorzugsweise alle wesentlichen Informationen enthalten, die für die Herstellung der Dialyselösung sowie für deren Verabreichung erforderlich sind, so dass das Risiko für Fehler etwa aufgrund falscher Verdünnung, der Verwechslung von Lösungsbeuteln etc. minimiert wird.

Vorzugsweise ist vorgesehen, dass der erste Konnektor und der zweite Konnektor voneinander lösbar sind, beispielsweise durch eine Brechverbindung.

Vorzugsweise handelt es sich bei dem ersten und bei dem zweiten Code jeweils um einen Barcode. Dieser ist vorzugsweise umlaufend ausgeführt, so dass es keine Rolle spielt, in welcher Orientierung sich der Konnektor beim Auslesen befindet.

Denkbar ist es, dass sich zwischen dem ersten und dem zweiten Konnektor eine Sollbruchstelle befindet. In diesem Fall wird zunächst der erste Code ausgelesen und sodann aus dem Konzentrat durch Zugeben von Verdünnungsmittel, insbesondere RO-Wasser (RO: Reverse Osmose) die Dialyselösung hergestellt. Ist dieser Vorgang abgeschlossen, wird der erste Konnektor abgebrochen und der zweite Konnektor in eine Aufnahme eines Dialysegerätes, vorzugsweise eines Peritonealdialysegerätes eingesteckt und dort ausgelesen. Damit liegt in dem Dialysegerät die Information vor, um welche Dialyselösung es sich handelt.

Vorzugsweise ist vorgesehen, dass die beiden Konnektoren hintereinander angeordnet sind, so dass nach der Herstellung der Dialyselösung die beiden Konnektoren und damit die beiden Codes voneinander getrennt werden können, so dass dann das durch den zweiten Code codierte Konzentratbehältnis, das die Dialyselösung enthält, direkt für die Behandlung zur Verfügung steht.

Der erste und der zweite Konnektor können durch eine Steck- oder Überwurfverbindung miteinander in Verbindung stehen. So ist es beispielsweise denkbar, dass nach der Herstellung der Dialyselösung auf oder in den ersten Konnektor der zweite Konnektor aufgesteckt wird. Wird das Konzentratbehältnis mit der fertigen Dialyselösung sodann mit dem Dialysegerät verbunden, wird der zweite und nicht der erste Code ausgelesen.

Vorzugsweise sind die beiden Konnektoren benachbart zueinander angeordnet.

Denkbar ist es, dass der erste Code räumlich weiter von dem Konzentratbehältnis entfernt angeordnet ist als der zweite Code. Nach der Herstellung der Dialyselösung kann der erste Code abgetrennt werden, so dass an dem Konzentratbehältnis nur der zweite Konnektor verbleibt, dessen Code sodann ausgelesen werden kann.

Die Konnektoren sind entweder unmittelbar an dem Konzentratbehältnis angeordnet oder, wenn die Konnektoren an einem Leitungsstück, insbesondere an einem Schlauch angeordnet sind, steht dessen Hohlraum mit dem Innenraum des Konzentratbehältnisses in Verbindung. Durch dieses Leitungsstück wird das Verdünnungsmittel in das Konzentratbehältnis geleitet und auch die Dialyselösung aus dem Konzentratbehältnis abgeführt.

Weiterhin kann eine Leitung vorgesehen sein, die sich zwischen dem oder den Konnektoren und dem Konzentratbehältnis erstreckt, wobei sich in der Leitung ein Verbindungsstück, insbesondere ein Luer-Konnektor befindet, durch das das Konzentratbehältnis von dem oder den Konnektoren trennbar ist. Dies ermöglicht es, dass das geleerte Konzentratbehältnis im Anschluss als Drainagebehältnis verwendet werden kann. Hierzu wird das Verbindungsstück, das beispielsweise als Steckverbindung ausgeführt sein kann, geöffnet, so dass der Konnektor von dem Konzentratbehältnis getrennt ist.

Das Konzentratbehältnis einschließlich der Konnektoren mit Codes können als komplette Einheit zur Verfügung gestellt werden bzw. getrennt geliefert werden. Durch die Verbindung der Einzelkomponenten von Konzentratbehältnis bzw. Mischbeutel vergrößert sich die Möglichkeit der Varianz für unterschiedlich konzentrierte Fertiglösungen und Beutel- bzw. Behältnisgrößen, ohne dass für jede mögliche Fertiglösung eine eigene Einheit (Konzentratbehältnis bzw. Mischbeutel) bevorratet werden muss.

Grundsätzlich kann zum Schutz vor Verkeimung bzw. Verunreinigung eine Desinfektionskappe für die Konnektoren vorgesehen sein.

Denkbar ist es, dass das Konzentratbehältnis starr ist. Grundsätzlich ist von der Erfindung jedoch auch umfasst, dass es sich um ein Behältnis mit flexiblen Wandungen handelt, d. h. um einen Beutel.

Auch eine Kombination dieser Ausführungsformen ist denkbar und von der Erfindung umfasst.

So ist es möglich, dass das Konzentratbehältnis im Sinne der vorliegenden Erfindung mehrere Behältnisse aufweist und zwar in Form eines Konzentrataufnahmebehältnisses, das das Konzentrat enthält, und eines Lösungsbehältnisses zur Aufnahme der Dialyselösung. Beide stehen in Fluidverbindung miteinander, so dass ein Lösungsmittel, insbesondere RO-Wasser zunächst mit dem Konzentrat in dem Konzentrataufnahmebehältnis vollständig gemischt werden kann und diese Mischung, d.h. die Dialyselösung sodann in das Lösungsbehältnis geleitet werden kann. Das Konzentrataufnahmebehältnis weist den ersten Konnektor mit dem ersten Code auf und das Lösungsbehältnis den zweiten Konnektor mit dem zweiten Code.

Der Begriff "Konzentratbehältnis" ist somit nicht zwingend dahingehend zu verstehen, dass genau ein Behältnis vorgesehen ist. Es können auch mehrere Behältnisse vorgesehen sein.

Dabei ist das Konzentrataufnahmebehältnis vorzugsweise als starrer Behälter (Cartridge) ausgeführt und/oder das Lösungsbehältnis als Beutel ausgebildet, der die gemischte Lösung, d.h. die Dialyselösung aufnimmt. Die feste Wandung der Cartridge ermöglicht es zu verhindern, dass Lösungsmittel bzw. unverdünntes Konzentrat in das Dialysegerät hinein gepumpt wird, da eine Volumenentnahme aus dem festen Behälter, welcher nicht vollständig gefüllt ist, nicht möglich ist. Zusätzlich kann bei dem Versuch der Entnahme der entsprechende Saugdruck als Indikator für einen Verschluss bzw. Nicht-Vermischung mit dem Leerbeutel bzw. mit dem Lösungsbehältnis herangezogen werden.

Die beiden Behältnisse können mittels eines Anschlussstücks verbunden werden. Durch die Verbindung beider Komponenten wird das Konzentrataufnahmebehältnis beutelseitig bzw. auf Seiten des Lösungsbehältnisses geöffnet.

Die Ausprägung des Einlasses bzw. der Auslassöffnung des Konzentrataufnahmebehältnisses kann das Vermischen der Lösung mit dem Konzentrat unterstützen.

Das Konzentrataufnahmebehältnis kann als Luftabscheider dienen.

Das Lösungsbehältnis, das vorzugsweise als Beutel ausgeführt ist, übernimmt in einer bevorzugten Ausgestaltung mehrere Funktionen: Mit Hilfe seines Verschlusssystems durchstößt es die Konnektorstelle am Cartridge bzw. am Konzentrataufnahmebehältnis, es nimmt das Gemisch, d.h. die Dialyselösung auf, es gibt die Dialyselösung während der Behandlung ab und es dient in der nächsten Behandlung als Drainagebeutel.

Das Konzentrataufnahmebehältnis ist vorzugsweise mit dem ersten Konnektor mit dem ersten Code und das Lösungsbehältnis mit dem zweiten Konnektor mit dem zweiten Code verbunden. Dabei können sich die beiden Konnektoren auf derselben Seite oder auf unterschiedlichen Seiten des Konzentrataufnahmebehältnisses befinden. Vorzugsweise ist das Lösungsbehältnis mitsamt dem zweiten Konnektor von dem Konzentrataufnahmebehältnis lösbar, beispielsweise indem der zweite Konnektor von dem Konzentrataufnahmebehältnis abgebrochen oder anderweitig abgetrennt wird.

Vorzugsweise handelt es sich bei dem Konzentratbehältnis um einen Einwegartikel.

Denkbar ist es, dass das Dialysegerät bzw. die Verdünnungseinrichtung eine Konnektorleiste aufweist, in der der oder die Konnektoren aufgenommen sind und dass ein starres Konzentratbehältnis von der Konnektorleiste mechanisch gestützt wird. Dies gilt vorzugsweise auch für das genannte starre Konzentrataufnahmebehältnis.

Die vorliegende Erfindung betrifft des Weiteren ein Dialysegerät, insbesondere ein Peritonealdialysegerät für die Durchführung einer Dialysebehandlung bzw. einer Peritonealdialysebehandlung, wobei das Dialysegerät wenigstens eine Aufnahme für den ersten Konnektor und/oder für den zweiten Konnektor eines Konzentratbehältnisses nach einem der Ansprüche 1 bis 12 sowie Lesemittel zum Auslesen der Informationen der auf dem oder den Konnektoren befindlichen Codes aufweist, wobei das Dialysegerät ausgebildet ist, in Abhängigkeit von dem Code Verdünnungsmittel in das Konzentratbehältnis abzugeben und/oder die Dialyselösung aus dem Behälter abzuführen.

Das Dialysegerät kann über eine Pumpeinrichtung verfügen, mittels derer das Verdünnungsmittel, insbesondere Wasser in das Konzentratbehältnis gefördert wird. Denkbar ist es, dass das Dialysegerät an eine Wasserversorgungsleitung angeschlossen wird und mittels der genannten Pumpvorrichtung die Dialyselösung hergestellt wird.

Dasselbe oder auch ein anderes Dialysegerät, insbesondere Peritonealdialysegerät verfügt über Lesemittel zum Auslesen des zweiten Codes, so dass die Dialyselösung durch die Pumpmittel in den Bauchraum des Patienten gefördert wird.

Unter dem Begriff "Dialysegerät" wird im Rahmen der vorliegenden Erfindung vorzugsweise ein Peritonealdialysegerät verstanden. Jedoch ist der Begriff bzw. die Erfindung nicht darauf beschränkt und umfasst auch andere Arten von Dialysegeräten. Bei dem Peritonealdialysegerät kann es um ein solches handeln, bei dem die Lösungen bzw. das Verdünnungsmittel durch Pumpen gefördert werden oder der Fluss gravimetrisch bewirkt wird, d.h. ohne den Einsatz einer Pumpe.

Die vorliegende Erfindung betrifft des Weiteren ein Verfahren zur Herstellung einer Dialyselösung, insbesondere einer Peritonealdialyselösung, wobei ein Konzentratbehältnis gemäß einem der Ansprüche 1 bis 12 mit dem Dialysegerät mit dem ersten Konnektor so verbunden wird, dass der erste Code durch das Dialysegerät ausgelesen wird und in Abhängigkeit des ersten Codes Verdünnungsmittel in das Konzentratbehältnis eingeleitet wird und dass im Anschluss daran, der erste Code entfernt oder überdeckt wird und das Konzentratbehältnis mit dem zweiten Konnektor mit demselben oder einem anderen Dialysegerät so verbunden wird, dass der zweite Code durch das Dialysegerät ausgelesen wird.

Denkbar ist es, dass nach dem Verdünnen des Konzentrats der erste Konnektor abgebrochen wird oder mit dem zweiten Konnektor verbunden wird oder durch den zweiten Konnektor überdeckt wird. Somit kann nach dem Verdünnen der zweite Konnektor ausgelesen werden, dessen Code Informationen zu der Dialyselösung enthält, die durch das Verdünnen des Konzentrats hergestellt ist.

An dieser Stelle wird darauf hingewiesen, dass die Begriff "ein" und "eine" nicht zwingend bedeuten, dass es sich um genau eines der fraglichen Elemente handelt, wenngleich dies eine bevorzugte Ausgestaltung der Erfindung darstellt. Vielmehr ist von der Verwendung dieser Begriffe auch die Mehrzahl der Elemente umfasst. Entsprechend gilt, dass die Verwendung eines Begriffs im Plural auch den Singular mit einschließt und umgekehrt, die Verwendung eines Begriffs im Singular auch die Mehrzahl des fraglichen Elementes mit umfasst.

Weitere Einzelheiten und Vorteile der Erfindung werden anhand eines in der Zeichnung dargestellten Ausführungsbeispiels näher erläutert. Es zeigen:
- Figur 1:: ein Konzentratbehältnis vor dem Anstecken des zweiten Konnektors an den ersten Konnektor,
- Figur 2:: ein Konzentratbehältnis mit zwei hintereinander angeordneten Konnektoren und einer dazwischen angeordneten Sollbruchstelle,
- Figur 3:: eine Ansicht eines Peritonealdialysegerätes mit mehreren angeschlossenen Beuteln und
- Figur 4:: ein Konzentratbehältnis gemäß Figur 2 mit einem in der Leitung angeordneten Verbindungsstück.
- Figur 5:: Ansichten unterschiedlicher Ausführungsformen von Konzentratbehältnissen, die ein Konzentrataufnahmebehältnis und ein Lösungsbehältnis aufweisen.

Es wird darauf hingewiesen, dass gleiche oder funktionsgleiche Elemente in den Figuren mit gleichen Bezugszeichen versehen sind.

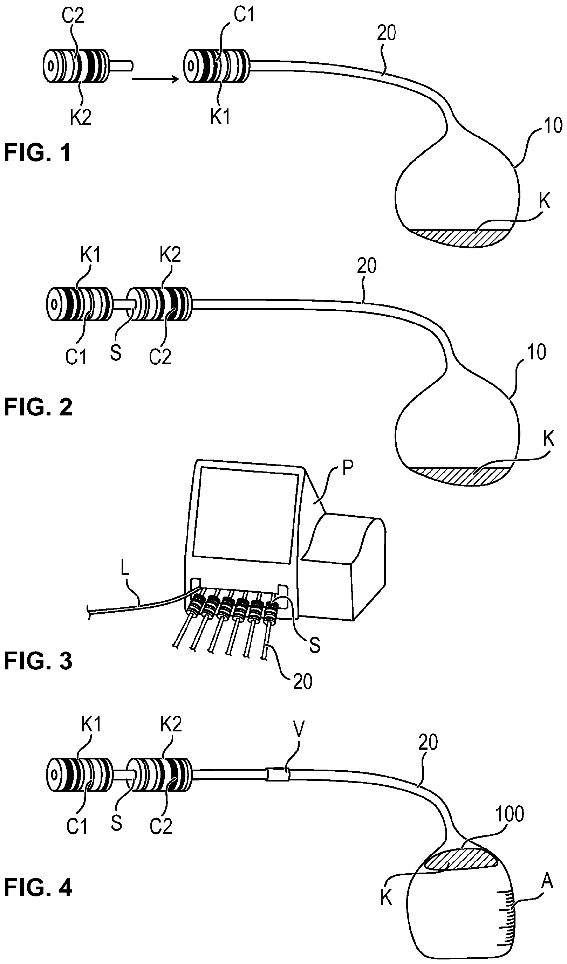

In dem Ausführungsbeispiel gemäß Figur 1 ist mit dem Bezugszeichen 10 das Konzentratbehältnis dargestellt, in dem sich ein flüssiges oder festes Konzentrat K befindet.

Das Konzentratbehältnis 10 ist mit einer Schlauchleitung 20 verbunden, an deren Ende sich der erste Konnektor K1 mit einem ersten Code C1 befindet.

In einer Verdünnungsstation, die beispielsweise durch ein Peritonealdialysegerät oder auch durch eine andere Einheit gebildet wird, wird Wasser durch den Schlauch 20 in das Innere des Konzentratbehältnisses 10 eingeleitet und das Konzentrat dementsprechend verdünnt. Die Verdünnungsstation liest den ersten Code C1 aus und nimmt die Verdünnung in Abhängigkeit davon vor. Nach der Verdünnung wird die Konnektorleiste der Verdünnungsstation geöffnet und der oder die Konnektoren K1 mit angeschlossenem Beutel 10 oder sonstigen Konzentratbehältnis 10 werden entnommen und durch einen Überwurf K2 mit einem Code C2 verschlossen. Der Code C2 trägt Informationen betreffend das verdünnte Konzentrat, d.h. der Dialyselösung.

Bei den Codes C1 und C2 handelt es sich um Barcodes. Auch eine andere Codierung ist möglich, z.B. eine unterschiedliche farbliche Codierung der Konnektoren.

Diese so codierten Beutel bzw. Konzentratbehältnisse können nun dem Patienten verabreicht werden.

In Figur 2 ist der Beutelanschluss bzw. der Konnektor um einen weiteren Konnektor erweitert. Dabei ist der näher zum Konzentratbehältnis angeordnete Konnektor K2 mit dem Code C2 durch eine Sollbruchstelle S mit dem weiter weg von dem Konzentratbehältnis angeordneten Konnektor K1 mit dem Code C1 verbunden.

Der beutelnahe Konnektor K2 bzw. dessen Code C2 enthält Informationen zu dem verdünnten Konzentrat, d.h. zu der Dialyselösung, der beutelferne Konnektor K1 bzw. dessen Code C1 enthält Informationen zu dem nicht verdünnten Konzentrat.

Nach der Verdünnung des Konzentrats K wird der Konnektor K1 abgebrochen so dass das Konzentratbehältnis mit dem Konnektor K2 nun für die Behandlung zur Verfügung steht.

Wie dies aus Figur 3 hervorgeht, dient in dem hier gezeigten Ausführungsbeispiel das Peritonealdialysegerät P als Verdünnungsstation, um fertige Lösungen für andere Peritonealdialysegeräte zur Verfügung zu stellen. Durch den Barcode C1 erkennt die Verdünnungsstation die Art des Konzentrats und gibt die benötigte Menge an Wasser zu.

Wie dies aus Figur 3 ersichtlich ist, können durch den Anschluss mehrerer Konzentratbehältnisse mit demselben Gerät gleichzeitig oder nacheinander mehrere Konzentratbehältnisse (hier sechs Konzentratbehältnisse) in einem Mischvorgang hergestellt werden.

Dazu wird die Patientenleitung L mit einer Lösungsmittelversorgung (z.B. RO-Wasser Anlage) verbunden. Nach dem Anmischen wird der Beutel bzw. das Konzentratbehältnis an der vorgesehenen Bruchstelle (vgl. Figur 2 und Figur 3, Bezugszeichen S) gelöst und steht nun sofort zur Verabreichung zur Verfügung, während weitere Gemische hergestellt werden können.

Eine denkbare Ausführungsform besteht darin, großvolumige Mengen anzumischen, um so mehrere Geräte versorgen zu können. Das verwendete Set steht nach dem Anmischen der Beutel bzw. Konzentratbehältnisse nicht mehr für die Behandlung zur Verfügung.

Weiterhin ist es dankbar, dem Konnektor ein Ventil hinzuzufügen, das ausschließlich das Hineinfördern von Flüssigkeit in den angeschlossenen Beutel ermöglicht. Ein versehentliches Ansaugen ist somit ausgeschlossen.

In dem Ausführungsbeispiel gemäß Figur 2 sind zu Beginn des Befüllens des Konzentratbehältnisses beide Konnektoren K1 und K2 an dem Konzentratbehältnis angeschlossen wie dies aus Figur 2 hervorgeht. Nach dem Befüllen des Beutels wird die Verbindung zwischen den Konnektoren K1 und K2 gelöst, z.B. durch Brechen.

Durch diesen Vorgang wird das Konzentratbehältnis zu einem eindeutig identifizierbaren Lösungsbeutel, der die Dialyselösung enthält.

Figur 4 zeigt ein Konzentratbehältnis, in dessen Schlauchleitung ein zusätzliches Verbindungsstück V angeordnet ist. Somit kann das Konzentratbehältnis nach der Behandlung ebenfalls als Drainagebeutel verwendet werden.

Mit dem Bezugszeichen A ist eine Volumenangabe in Form von Messstrichen am Beutel bzw. Konzentratbehältnis gekennzeichnet. Diese ermöglicht eine zusätzliche Sichtkontrolle der gefüllten Beutel nach Entnahme aus der Füllstation, etwa dahingehend, dass der Beutel bis zu einem bestimmten Niveau gefüllt sein muss.

Denkbar ist es, das Konzentratbehältnis mit einem zusätzlichen Griff zu versehen, der dessen Transport erleichtert, insbesondere wenn es sich um eine größere Flüssigkeitsmenge handelt.

In dem Ausführungsbeispiel gemäß Figur 4 liegt das Konzentrat verpackt in dem Konzentratbehältnis vor. Diese Verpackung ist mit dem Bezugszeichen 100 gekennzeichnet. Sie degradiert beim Kontakt mit dem Verdünnungsmittel und befindet sich vorzugsweise am Einlauf des Konzentratbehältnisses. Somit kann eine optimale Durchmischung mit der Lösung während des Befüllens erreicht werden.

In einer weiteren denkbaren Ausgestaltung der Erfindung ist das Konzentrat mit einem unschädlichen Farbstoff eingefärbt. Dies ermöglicht die Überprüfung, ob das Konzentrat und das Verdünnungsmittel ausreichend gemischt sind.

Die vorliegende Erfindung bietet in einer vorteilhaften Ausgestaltung unter anderem die im Folgenden genannten Vorteile:
Ein Lösungsbeutel bzw. das Konzentratbehältnis kann direkt nach der Fertigstellung vom Verdünnungsgerät entnommen werden, während die nächsten Beutel bzw. Konzentratbehältnisse befüllt werden.

Die Lösungsbeutel bzw. Konzentratbehältnisse können zeitlich gestaffelt verdünnt und entnommen werden, um eine möglichst optimale Nutzung des Verbrauchszeitraums zu ermöglichen.

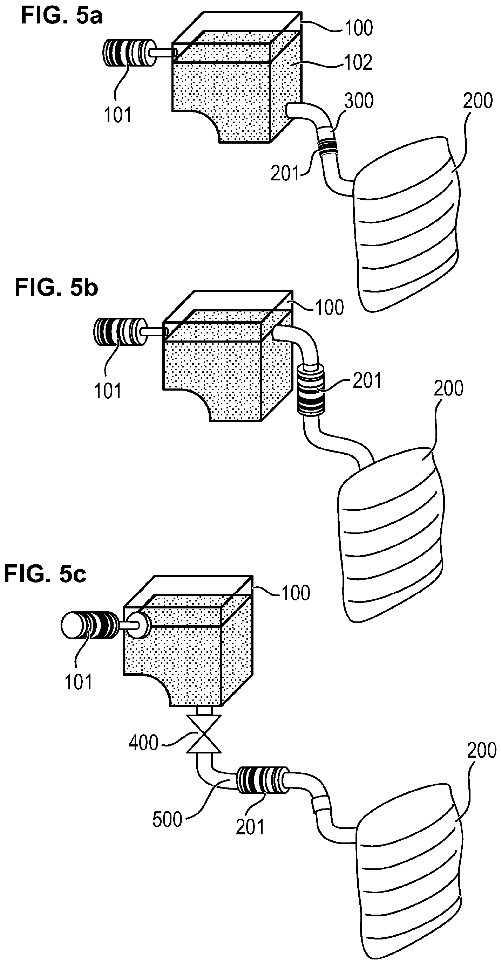

Figur 5 a) zeigt eine Ausführungsform, bei der das Konzentratbehältnis zwei Behältnisse aufweist, nämlich zum einen das Konzentrataufnahmebehältnis 100, das mit dem ersten Konnektor 101 mit dem ersten Code und das mit diesem in Fluidverbindung stehende Lösungsbehältnis 200 mit dem zweiten Konnektor 201. In dem Konzentrataufnahmebehältnis 100 befindet sich das Konzentrat 102. Durch das Anschlussstück 300 werden beide Behältnisse miteinander verbunden. Durch diese Verbindung wird das Konzentrataufnahmebehältnis 100 zum Beutel 200 hin geöffnet.

Figur 5 b) zeigt eine Ausführungsform, bei dem der Auslass des Konzentrataufnahmebehältnisses 100 in einem Barcodeabschnitt bzw. Konnektor 201 mündet, an dem das Beutelsegment angeschlossen werden kann.

Aus Figur 5 c) ist ersichtlich, dass ein Ventil 400 vorgesehen sein kann, das ausschließlich das Hineinförden von Flüssigkeit über das Cartridge 100 in den angeschlossenen Lösungsbeutel 200 ermöglicht. Das Ansaugen des Konzentrats ist somit konstruktiv unterbunden. Nach dem Befüllen des Lösungsbeutels wird die Verbindung zwischen den Behältnissen bei 500 unterbrochen.

Zu Beginn des Füllens wird ein universeller Leerbeutel am Cartridge 100 angeschlossen. Nach dem Befüllen des Beutels wird die Verbindung zwischen Cartridge 100 und dem Barcodeabschnitt 201 gelöst. Durch diesen Vorgang wird der universeile Leerbeutel zu einem eindeutigen Lösungsbeutel, der nun mit einem zweiten Cycler verabreicht werden kann.

Somit kann ein Lösungsbeutel direkt nach der Fertigstellung vom Verdünnungsgerät entnommen werden, während die nächsten Beutel befüllt werden. Des Weiteren können Lösungsbeutel zeitlich gestaffelt verdünnt und entnommen werden, um eine möglichst optimale Nutzung des Verbrauchszeitraums zu ermöglichen.

Im Folgenden werden einige Szenarien vorgestellt, die exemplarischer Natur sind und die die Erfindung nicht beschränken.

### Szenario A:

Der Cycler, d.h. das Peritonealdialysegerät wird mit einer Lösungsmittelversorgung verbunden, ähnlich wie im HD Umfeld, dazu wird ein Beutelanschluss des Sets (Port) benötigt.

Die weiteren Ports können mit Cardridges besetzt werden. Die Cardridges werden mit den Leerbeuteln verbunden typischerweise 6l.

Nach Erzeugung des Gemisches kann der Behandlungsablauf unmittelbar gestartet werden.

### Szenario B:

Der Cycler wird mit einer Lösungsmittelversorgung verbunden. Dazu wird ein Beutelanschluss des Sets (Port) benötigt.

Nur ein Port wird mit dem Konzentrat besetzt. Das Cardridge wird mit einem Beutel verbunden, der das Volumen einer kompletten Behandlung aufnehmen kann.

Dies war bisher nicht möglich, da der Patient diesen gefüllten Beutel nicht transportieren muss. Eine solche Vorgehensweise reduziert die Kosten und Abfallmenge des Einwegartikels.

### Szenario C:

Ebenso ist es möglich, das Verfahren mit regulärem Lösungsbeutel zu kombinieren, welche innerhalb der Behandlung genutzt werden können.

### Szenario D:

Die derzeitige Patientenleitung, wird mit einer Lösungsmittelversorgung verbunden. Somit könnten in einem ersten Schritt bis zu sechs Beutel angemischt werden.

In einem zweiten Schritt wird die angemischte Lösung ohne Wechsel des Sets dem Patienten verabreicht.

### Szenario E:

Der Cycler fungiert als Verdünnungsstation um die Lösungen für andere Cycler bereitzustellen. So wäre es möglich sechs Beutel in einem Mischvorgang herzustellen.

Dazu wird die derzeitige Patientenleitung mit einer Lösungsmittelversorgung verbunden. Nach dem Anmischen wird der Beutel von dem Cardridge gelöst und steht nun zur Verabreichung zur Verfügung. Eine vorteilhafte Auslegung des System besteht darin großvolumige Mengen anzumischen. Da das Set, welches im Mischgerät benutzt wurde, für eine Behandlung nicht zur Verfügung steht. Ein dafür geeignetes Cardridge ist in Abbildung 5b zu sehen.

## Patentansprüche

1. Konzentratbehältnis (10) mit einem Konzentrat (K), das ausgebildet ist, nach seiner Verdünnung mit einem Verdünnungsmittel eine Dialyselösung zu bilden, wobei
das Konzentratbehältnis (10) mit einem ersten Konnektor (K1) in Verbindung steht, der einen ersten Code (C1) aufweist, der eine Information des Konzentrats (K) enthält, und
das Konzentratbehältnis (10) mit einem zweiten Konnektor (K2) in Verbindung steht, der einen sich zum ersten Code (C1) unterscheidenden zweiten Code (C2) aufweist, der eine Information der Dialyselösung enthält,
**dadurch gekennzeichnet, dass**
dass sowohl der erste Konnektor (K1) als auch der zweite Konnektor (K2) unmittelbar an dem Konzentratbehältnis (10) angeordnet sind oder an lediglich einem Leitungsstück (20) angeordnet sind, dessen Hohlraum mit dem Innenraum des Konzentratbehältnisses (10) in Verbindung steht.

2. Konzentratbehältnis (10) nach Anspruch 1, **dadurch gekennzeichnet, dass** der erste Konnektor (K1) und der zweite Konnektor (K2) voneinander lösbar sind.

3. Konzentratbehältnis (10) nach Anspruch 1 oder 2, **dadurch gekennzeichnet, dass** es sich bei dem ersten Code (C1) und bei dem zweiten Code (C2) jeweils um einen Barcode oder um eine farbige Codierung handelt.

4. Konzentratbehältnis (10) nach einem der vorhergehenden Ansprüche, **dadurch gekennzeichnet, dass** sich zwischen dem ersten Konnektor (K1) und dem zweiten Konnektor (K2) eine Sollbruchstelle (S) befindet.

5. Konzentratbehältnis (10) nach einem der vorhergehenden Ansprüche, **dadurch gekennzeichnet, dass** der erste Konnektor (K1) und der zweite Konnektor (K2) durch eine Steck- oder Überwurfverbindung miteinander in Verbindung stehen.

6. Konzentratbehältnis (10) nach einem der vorhergehenden Ansprüche, **dadurch gekennzeichnet, dass** erste Code (C1) räumlich weiter weg von dem Konzentratbehältnis (10) angeordnet ist als der zweite Code (C2).

7. Konzentratbehältnis (10) nach einem der vorhergehenden Ansprüche, **dadurch gekennzeichnet, dass** eine Leitung vorgesehen ist, die sich zwischen dem oder den Konnektoren (K1, K2) und dem Konzentratbehältnis (10) erstreckt, wobei sich in der Leitung ein Verbindungsstück befindet, durch das das Konzentratbehältnis (10) von dem oder den Konnektoren (K1, K2) trennbar ist.

8. Konzentratbehältnis (10) nach einem der vorhergehenden Ansprüche, **dadurch gekennzeichnet, dass** das Konzentratbehältnis (10) aus einem einzigen Behältnis, vorzugsweise aus einem einzigen Beutel besteht.

9. Konzentratbehältnis (10) nach einem der Ansprüche 1 bis 7, **dadurch gekennzeichnet, dass** das Konzentratbehältnis (10) mehrere Behältnisse aufweist und ein Konzentrataufnahmebehältnis (100), das das Konzentrat (K) enthält, und ein Lösungsbehältnis (200) zur Aufnahme der Dialyselösung aufweist.

10. Konzentratbehältnis (10) nach Anspruch 9, **dadurch gekennzeichnet, dass** das Konzentrataufnahmebehältnis (100) als starrer Behälter ausgeführt ist und/oder dass das Lösungsbehältnis (200) als Beutel ausgeführt ist.

11. Konzentratbehältnis (10) nach Anspruch 9 oder 10, **dadurch gekennzeichnet, dass** das Konzentrataufnahmebehältnis (100) mit dem ersten Konnektor (K1) mit dem ersten Code (C1) und das Lösungsbehältnis (200) mit dem zweiten Konnektor (K2) mit dem zweiten Code (C2) verbunden ist.

12. Verfahren zur Herstellung einer Dialyselösung, **dadurch gekennzeichnet, dass** ein Konzentratbehältnis (10) gemäß einem der Ansprüche 1 bis 11 mit einem Dialysegerät (P) mit dem ersten Konnektor (K1) so verbunden wird, dass der erste Code (C1) durch das Dialysegerät (P) ausgelesen wird und in Abhängigkeit des ersten Codes (C1) Verdünnungsmittel in das Konzentratbehältnis (10) eingeleitet wird und dass im Anschluss daran, der erste Konnektor (K1) entfernt, überdeckt oder mit dem zweiten Konnektor (K2) verbunden wird und das Konzentratbehältnis (10) mit dem zweiten Konnektor (K2) mit demselben oder einem anderen Dialysegerät (P) so verbunden wird, dass der zweite Code (C2) durch das Dialysegerät (P) ausgelesen wird.

13. Verfahren nach Anspruch 12, **dadurch gekennzeichnet, dass** nach dem Verdünnen des Konzentrats (K) der erste Konnektor (K1) abgebrochen wird.

## Claims

1. A concentrate container (10) having a concentrate (K) that is configured to form a dialysis solution after its dilution with a diluting agent, wherein
the concentrate container (10) is in communication with a first connector (K1) having a first code (C1) that contains a piece of information on the concentrate (K); and
the concentrate container (10) is furthermore in communication with a second connector (K2) having second code (C2) differing from the first code (C1), and containing a piece of information on the dialysis solution,
**characterized in that**
both the first connector (K1) and the second connector (K2) are arranged directly at the concentrate container (10) or are only arranged at a line piece (20) whose hollow space is in communication with the inner space of the concentrate container (10).

2. A concentrate container (10) in accordance with claim 1, **characterized in that** the first connector (K1) and the second connector (K2) are releasable from one another.

3. A concentrate container (10) in accordance with claim 1 or claim 2, **characterized in that** the first code (C1) and the second code (C2) are each barcodes or color codes.

4. A concentrate container (10) in accordance with one of the preceding claims, **characterized in that** a predetermined breaking point (S) is located between the first connector (K1) and the second connector (K2).

5. A concentrate container (10) in accordance with one of the preceding claims, **characterized in that** the first connector (K1) and the second connector (K2) are connected to one another by a plug-in connection or a cap connection.

6. A concentrate container (10) in accordance with one of the preceding claims, **characterized in that** the first code (C1) is arranged spatially further away from the concentrate container (10) than the second code (C2).

7. A concentrate container (10) in accordance with one of the preceding claims, **characterized in that** a line is provided that extends between the connector or connectors (K1, K2) and the concentrate container (10), with a connection piece being located in the line by which the concentrate container (10) can be separated from the connector or connectors (K1, K2).

8. A concentrate container (10) in accordance with one of the preceding claims, **characterized in that** the concentrate container (10) comprises a single container, preferably a single bag.

9. A concentrate container (10) in accordance with one of the claims 1 to 7, **characterized in that** the concentrate container (10) has a plurality of containers and has a concentrate reception container (100) that contains the concentrate (K) and a solution container (200) for receiving the dialysis solution.

10. A concentrate container (10) in accordance with claim 9, **characterized in that** the concentrate reception container (100) is configured as a rigid container; and/or **in that** the solution container (200) is configured as a bag.

11. A concentrate container (10) in accordance with claim 9 or claim 10, **characterized in that** the concentrate reception container (100) is in communication with the first connector (K1) having the first code (C1) and the solution container (200) is in communication with the second connector (K2) having the second code (C2).

12. A method of preparing a dialysis solution, **characterized in that** a concentrate container (10) in accordance with one of the claims 1 to 11 is connected to a dialysis machine (P) by the first connector (K1) such that the first code (C1) is read by the dialysis machine (P) and diluting agent is introduced into the concentrate container (10) in dependence on the first code (C1) and such that the first connector (K1) is subsequently removed, covered or connected to the second connector (K2) and the concentrate container (10) is connected to the same dialysis machine (P) or to a different dialysis machine (P) by the second connector (K2) such that the second code (C2) is read by the dialysis machine (P).

13. A method in accordance with claim 12, characterized the first connector (K1) is broken off after the dilution of the concentrate (K).

## Revendications

1. Récipient de concentré (10) comprenant un concentré (K), qui est conçu pour former, après sa dilution avec un diluant, une solution de dialyse, dans lequel
le récipient de concentré (10) est relié à un premier connecteur (K1), qui comporte un premier code (C1), qui contient une information relative au concentré (K), et
le récipient de concentré (10) est relié à un second connecteur (K2), qui comporte un second code (C2) différent du premier code (C1), qui contient une information relative à la solution de dialyse,
**caractérisé en ce que**
aussi bien le premier connecteur (K1) que le second connecteur (K2) sont disposés directement sur le récipient de concentré (10) ou sont disposés sur uniquement un morceau de tubulure (20) dont l'espace vide est relié à l'espace intérieur du récipient de concentré (10).

2. Récipient de concentré (10) selon la revendication 1, **caractérisé en ce que** le premier connecteur (K1) et le second connecteur (K2) peuvent être détachés l'un de l'autre.

3. Récipient de concentré (10) selon la revendication 1 ou 2, **caractérisé en ce que** le premier code (C1) et le second code (C2) sont chacun un code à barres ou un codage couleur.

4. Récipient de concentré (10) selon l'une des revendications précédentes, **caractérisé en ce qu'**un point destiné à la rupture (S) se trouve entre le premier connecteur (K1) et le second connecteur (K2).

5. Récipient de concentré (10) selon l'une des revendications précédentes, **caractérisé en ce que** le premier connecteur (K1) et le second connecteur (K2) sont reliés l'un à l'autre par une liaison par emboîtement ou par raccord union.

6. Récipient de concentré (10) selon l'une des revendications précédentes, **caractérisé en ce que** le premier code (C1) est disposé plus éloigné dans l'espace du récipient de concentré (10) que le second code (C2).

7. Récipient de concentré (10) selon l'une des revendications précédentes, **caractérisé en ce qu'**une tubulure est prévue, qui s'étend entre le ou les connecteurs (K1, K2) et le récipient de concentré (10), une pièce de liaison se trouvant dans la tubulure, par laquelle le récipient de concentré (10) peut être séparé du ou des connecteurs (K1, K2).

8. Récipient de concentré (10) selon l'une des revendications précédentes, **caractérisé en ce que** le récipient de concentré (10) est constitué d'un seul récipient, de préférence d'une seule poche.

9. Récipient de concentré (10) selon l'une des revendications 1 à 7, **caractérisé en ce que** le récipient de concentré (10) comporte plusieurs récipients et comporte un récipient de réception de concentré (100), qui contient le concentré (K), et un récipient de solution (200) pour recevoir la solution de dialyse.

10. Récipient de concentré (10) selon la revendication 9, **caractérisé en ce que** le récipient de réception de concentré (100) est réalisé sous la forme d'un contenant rigide et/ou **en ce que** le récipient de solution (200) est réalisé sous la forme d'une poche.

11. Récipient de concentré (10) selon la revendication 9 ou 10, **caractérisé en ce que** le récipient de réception de concentré (100) est relié au premier connecteur (K1) avec le premier code (C1) et le récipient de solution (200) au second connecteur (K2) avec le second code (C2).

12. Procédé de fabrication d'une solution de dialyse, **caractérisé en ce qu'**un récipient de concentré (10) selon l'une des revendications 1 à 11 est relié à un appareil de dialyse (P) avec le premier connecteur (K1) de telle manière que le premier code (C1) est lu par l'appareil de dialyse (P) et qu'un diluant est introduit dans le récipient de concentré (10) en fonction du premier code (C1) et **en ce que**, ensuite, le premier connecteur (K1) est retiré, recouvert ou relié au second connecteur (K2) et le récipient de concentré (10) est relié avec le second connecteur (K2) au même ou à un autre appareil de dialyse (P) de telle manière que le second code (C2) est lu par l'appareil de dialyse (P).

13. Procédé selon la revendication 12, **caractérisé en ce que**, après la dilution du concentré (K), le premier connecteur (K1) est cassé.
